# EUROPEAN PATENT SPECIFICATION

(11) **EP 0 340 862 B1**
(45) Date of publication and mention of the grant of the patent: **24.11.1993**
(21) Application number: 89201110.7
(22) Date of filing: 01.05.1989
(51) Int. Cl.: C07C 15/085, C07C 2/66, C07C 6/12, B01J 21/12

(54) **Method for preparing cumene**
Verfahren zur Herstellung von Cumol
Procédé pour la préparation du cumène

(30) Priority: 06.05.1988 IT 2049588
(43) Date of publication of application: 08.11.1989
(73) Proprietor: ENICHEM SYNTHESIS S.p.A., 90139 Palermo (IT); ENIRICERCHE S.p.A., 20121 Milano (IT)
(72) Inventor: Cavani, Fabrizio, I-41100 Modena (IT); Arrigoni, Virginio, I-20138 Milan (IT); Bellussi, Giuseppe, I-29100 Piacenza (IT)
(74) Representative: Fusina, Gerolamo

(56) References cited:
- EP-A- 0 160 145
- US-A- 366 790
- US-A- 2 589 057
- US-A- 4 622 311
- PREPRINTS, GENERAL PAPERS, SYMPOSIA, DIVISION OF PETRO- -LEUM CHEMISTRY, INC. AMERICAN CHEMICAL SOCIETY, vol. 22, no. 1, February 1977 E.F.HARPER "Alkylation of benzene wirt propylene over a crystalline alumina silicate" pages 1084-1098

## Description

This invention relates to a catalytic process for preparing cumene by alkylating benzene with propylene, or by transalkylating diisopropylbenzenes in the presence of benzene, and, also, to a process for preparing an alkylation/transalkylation catalyst.

GN-A-769 383, US-A-2 887 520 and US-A-4 912 279 disclose several hydrocarbon modification processes, including alkylation runs, but are impaired by drawbacks inherent in corrosion problems and disposal of the spent catalyst. To overcome such shortcomings, US-A-4 459 425 and US-A-4 395 372 have suggested zeolites, more particularly zeolite Y, as the catalyst for the reactions of the kind referred to above.

Zeolite catalysts, however, have a low selectivity towards the useful reaction product, due to a large production of polyalkylation products and oligomerization of the alkylating agent.

EP-A-0 160 145 discloses an aromatics alkylation process catalyzed by an amorphous silica and alumina gel with a pore diameter in the order of 5 nm to 50 nm (50 A to 500 A), having a silica to alumina ratio of from 1:1 to 10:1. Catalysts of this kind are fairly active with C₄-C₂₀ alkylating agents.

Finally, US-A1-B 366 790 discloses a cumene preparation process which is carried out by reacting benzene with propylene in the liquid phase in the presence of an amorphous aluminium silicate at a comparatively high temperature, that is, a temperature of from 249°C to 316°C.

The technical problem was thus to prepare cumene by the reactions referred to above with an unexpectedly high selectivity towards the useful reaction product and adopting lower reaction temperatures.

The present invention, therefore, provides a catalytic process for preparing cumene by alkylating benzene with propylene, or by transalkylating diisopropylbenzenes in the presence of benzene, either reaction being catalyzed by an X-ray-amorphous silica-alumina gel having an SiO₂/Al₂O₃ of from 50:1 to 300:1, comprising the step of reacting benzene with propylene at a temperature of from 100°C to 250°C under a pressure of from 2 MPa to 5 Mpa (20 to 50 ata), the molar ratio of benzene to propylene being from 2:1 to 30:1, or transalkylating diisopropylbenzenes in the presence of benzene at a temperature of from 150°C to 250°C under a pressure of from 1 Mpa to 4 MPa (10 to 40 ata), the molar ratio of benzene to the diisopropylbenzenes being from 10:1 to 40:1, in the presence of an X-ray-amorphous silica-alumina gel having a surface area of from 500 m²/g to 1000 m²/g, an overall pore volume of from 0,3 ml/g to 0, 6 ml/g, and a mean pore diameter of from 1 nm (10 A), said silica-alumina gel being free of pores having a diameter greater than 3 nm (30 A).

According to another aspect, the present invention also provides a process for preparing an X-ray-amorphous silica-alumina gel having a surface area of from 500 m²/g to 1000 m²/g, an overall pore volume of from 0,3 ml/g to 0, 6 ml/g, and a mean pore diameter of from 1 nm (10 A), said silica-alumina gel being free of pores having a diameter greater than 3 nm (30 A) for use as an alkylation or transalkylation catalyst, comprising the steps of:
(a) Preparing an aqueous solution of a tetraalkyl hydroxide (TAA-OH), a water soluble Al compound capable of being hydrolyzed to Al₂O₃ and a soluble Si compound capable of being hydrolyzed to SiO₂, the molar ratios of the solutes being:
   SiO₂ to Al₂O₃: from 30:1 to 500:1
   TAA-OH to SiO₂: from 0,005:1 to 0,2:1
   H₂O to SiO₂ from 5:1 to 40:1;
(b) Heating said aqueous solution to gel it;
(c) Drying the as-obtained gel, and
(d) Calcining the dried gel initially in an inert atmosphere and finally in an oxidizing atmosphere.

Preferred aluminium trialkyl hydroxides (trialkoxides) for step (a) are Al tri-n.propoxide or Al tri-isopropoxide.

A preferred tetraalkyl silicate for stage (a) is tetraethylsilicate.

A convenient temperature for step (a) is from 20°C to 25°C.

According to a preferred embodiment of the catalyst preparation process, the molar ratios of the solutes are:
SiO₂ to Al₂O₃: from 50:1 to 300:1
TAA-OH to SiO₂: from 0,005:1 to 0,2:1
H₂O to SiO₂ from 10:1 to 25:1.

Step (b) is preferably conducted at a temperature of from 50°C to 70°C, and a still preferred temperature is 60°C.

The drying step (c) is carried out at a temperature not above 150°C and stage (c) can be conveniently started by spray-drying.

The calcining step (d) is conveniently conducted at a temperature of from 550°C to 700°C and is carried out initially in a nitrogen environment and completed in the presence of air.

The silica-alumina gel thus obtained, having the properties reported in the foregoing, is extremely active in the alkylation of aromatics with olefins (or their precursors), and furthermore it is particularly selective in the alkylation of benzene with propylene.

Consequently such silica and alumina gel has the inherent advantages of zeolite Y such as the high activity and compared with these is more selective towards the monoalkylation product cumene (isopropylbenzene) for equal reactant ratios.

The silica and alumina gel used as catalyst can be mixed with suitable metal oxides acting as binders. Suitable oxides for this purpose are aluminas, silicas, or titanium, magnesium or zirconium oxides. The silica and alumina gel and the binder can be mixed in weight ratios of between 50/50 and 95/5, and preferably between 70/30 and 90/10. The two components can be mixed by conventional methods and the mixture is conveniently consolidated into the final desired form, such as extrusions or granules. By operating in this manner the catalyst can be given improved mechanical properties.

The alkylation reaction can be conducted batchwise, semicontinuously or preferably continuously The reaction is preferably carried out continuously in a flow reactor at a temperature of between 100°C and 250°C, preferably between 110°C and 200°C, at a pressure of between 2 MPa and 5 MPa (20 and 50 ata), preferably of 3 MPa (30 ata), and with the reactants (benzene and propylene) being fed at a WHSV of between 0.1 and 50 hour⁻¹.

The reaction can be conducted in the gaseous phase, however it is preferable to operate in the liquid or mixed (liquid-vapour) phase. This minimizes carbon formation, so extending the catalyst life and giving improved selectivity.

The molar feed ratio of benzene to propylene can generally vary from 2/1 to 30/1 but it is preferable to operate within the range of 4/1 to 15/1. Within this range the catalyst life (time before regeneration) can be kept long, and the formation of higher molecular weight compounds (polyalkylates and propylene oligomers) can be minimized.

The alkylation reaction is exothermic and therefore under industrial conditions the temperature is controlled preferably by feeding cold benzene or inert paraffins (such as propane) at various levels of the catalyst bed.

The described silica and alumina gel is also active in the transalkylation of diisopropylbenzenes in the presence of benzene.

These diisopropylbenzenes can be the by-products of the alkylation of benzene with propylene. The molar ratio of benzene to diisopropylbenzenes can conveniently vary from 10/1 to 40/1 and the transalkylation reaction can be conducted at a temperature of between 150 and 250°C at a pressure of between 10 and 40 ata and, in the case of a continuous process, with a reactant feed rate in terms of WHSV of between 1 and 30 hour⁻¹. The transalkylation reaction can be conveniently conducted in the liquid phase.

In the experimental examples given hereinafter a silica and alumina gel is used having the following characteristics:
- amorphous to X-rays (analysis conducted on powder by Philips vertical goniometer using Cu K α radiation);
- molar SiO₂/Al₂O₃ ratio 100/1
- surface area = 800 m²/g (measured by Carlo Erba Sorptomatic 1800 apparatus);
- porosity = 0.44 ml/g, mean pore diameter 1 nm (10 Å), absence of pores with diameter exceeding 3 nm (30 Å) (values determined by Carlo Erba Sorptomatic 1800 apparatus).

This silica and alumina gel was prepared by the following procedure.

2.0 g of aluminium tri-isopropylate are dissolved in 34 g of 30.6 weight% tetrapropyl ammonium hydroxide (TPA-OH), and 162 g of demineralized water are added.

These operations are conducted at ambient temperature (about 20°C).

The solution obtained is heated to 60°C and 104 g of tetraethyl silicate (TES) are added while stirring. The mixture obtained has the following molar ratios:

| | |
|---|---|
| SiO₂/Al₂O₃ | 100/1 |
| TAA-OH/SiO₂ | 0.10/1 |
| H₂O/SiO₂ | 21/1. |

The mixture is kept stirred at 60°C and after 30 minutes a homogeneous gel is obtained and dried in a Rotavapor flask in an air stream with a bath temperature-controlled at 90°C, followed by drying in an oven at 100°C.

The dried gel is calcined at 550°C for 3 hours in a nitrogen stream and then for 10 hours in an air stream.

In this manner 30 g of silica and alumina gel are obtained with a quantitative yield with respect to the initially fed silicon and aluminium, and having the said characteristics.

The experimental examples given hereinafter further illustrate the invention.

In these examples alkylation tests on benzene with propylene and transalkylation tests on diisopropylbenzenes in the presence of benzene are described using the aforesaid silica and alumina gel catalyst.

For comparison purposes alkylation tests on benzene with propylene are described using zeolite Y of the known art.

### EXAMPLE 1

20.0 g of silica and alumina gel having the characteristics given in the description are fed into an autoclave together with 300 ml of benzene and 9.8 g of propylene. The molar ratio of feed benzene to propylene is therefore 14.5/1.

The autoclave is pressurised with nitrogen to obtain a total pressure of 4 MPa (40 ata) at the reaction temperature, which is fixed at 160°C. The mass is kept stirred for one hour after which the reactor contents are discharged and analysed chromatographically by means of a Hewlett Packard gas chromatograph with an QV 101 wide bore column and an 80°C-240°C programmed temperature.

The results of this analysis are given in Table 1, in which the headings have the following meanings:
C₆ % conv. = percentage conversion or benzene
C₃ % conv. = percentage conversion of propylene
C₆ % sel. = molar percentage selectivity of benzene converted into cumene
C₃ % sel. = molar percentage selectivity of propylene converted into cumene.

### EXAMPLE 2 (comparison)

Example 1 is repeated using as catalyst a protonic zeolite Y produced commercially by Union Carbide (Linde LZ-Y 62; 1,6 mm (1/16") extrusions). The results of this test are given in Table 1.

### EXAMPLE 3

The procedure of Example 1 is followed using the same silica and alumina gel catalyst, but with the following conditions:
temperature 150°C, pressure 3 MPa (30 ata) molar ratio of feed benzene to propylene 8.0/1 (300 ml benzene fed) and reaction time 1.5 hours.

The results of this test are given in Table 1.

### EXAMPLE 4 (comparison)

The procedure of Example 3 is followed using as catalyst a commercial zeolite Y of Toyo Soda (TSZ 360 HUD, in extrusion form). The results of this test are given in Table 1.

### EXAMPLE 5

The procedure of Example 1 is followed using the same silica and alumina gel catalyst, but with the following conditions:
temperature 150°C, pressure 1,05 MPa (10.5 ata) (autogenous system pressure at the stated temperature), molar ratio of feed benzene to propylene 8.0/1 and reaction time 2 hours. The results of this test are given in Table 1.

### EXAMPLE 6 (comparison)

The procedure of Example 5 is followed using the zeolite Y of Example 2 as catalyst. The results are given in Table 1.

### EXAMPLE 7 (comparison)

The procedure of Example 5 is followed using the zeolite Y of Example 4. The results are given in Table 1.

**TABLE 1**

| EXAMPLE | C₆ % CONV. | C₃ % CONV. | C₆ % SEL. | C₃ % SEL. |
|---|---|---|---|---|
| 1 | 4.8 | 72.1 | 95.7 | 91.7 |
| 2 | 5.3 | 85.2 | 93.2 | 87.2 |
| 3 | 8.5 | 73.8 | 91.3 | 83.4 |
| 4 | 9.0 | 82.2 | 88.3 | 78.7 |
| 5 | 8.3 | 70.4 | 92.7 | 86.3 |
| 6 | 8.1 | 71.6 | 90.0 | 81.4 |
| 7 | 8.0 | 71.5 | 89.3 | 80.3 |

Besides a high activity, better selectivity will be noted in the case of the examples conducted according to the invention than in those conducted with zeolite Y catalysts.

These better selectivity values derive from the smaller quantity of diisopropylbenzene by-products and the absence or virtual absence of butylbenzenes and pentylbenzenes. The diisopropylbenzenes form by consecutive alkylation reactions between benzene and propylene.

The butylbenzenes and pentylbenzenes present as by-products in the examples conducted using zeolite Y as catalyst presumably form following oligomerization of the propylene to nonene, cracking of said nonene to C₅ and C₄ olefins and benzene alkylation by these olefins.

### EXAMPLE 8

The autoclave of Example 1 is used for a transalkylation test using silica and alumina gel with the characteristics given in the description. Specifically, 20 g of said catalyst are used, feeding into the reactor 256 g of benzene and 17 g of diisopropylbenzenes (molar ratio of the two reactants 31/1). The reaction conditions are: pressure 3 MPa (30 ata), temperature 250°C, reaction time 2 hours.

Analysis of the reaction products indicates a diisopropylbenzene conversion of 86% with a selectivity towards cumene of 90%.

### EXAMPLE 9

The procedure of Example 8 is repeated except that the reaction temperature is fixed at 210°C. In this case the diisopropylbenzene conversion is 76% with a cumene selectivity of 90%.

### EXAMPLE 10

The alkylation reaction in the presence of silica and alumina gel having the characteristics given in the description is conducted in a vertical flow reactor. The reactor is formed from a 36 mm diameter tube of the height of 900 mm. A multiple thermocouple is inserted longitudinally to measure the catalyst bed temperature at various levels. The reactor is immersed in a bath of fluidized alumina to remove the heat of reaction.

In this example the silica and alumina gel catalyst is mixed with γ-alumina in a weight ratio of 100:25 and formed into 1/8" diameter extrusions of average length 8 mm. The reaction conditions are:
temperature 160°C, pressure 4 MPa (40 ata), molar ratio of feed benzene to propylene 14.5/1, feed rate of organic phase (benzene + propylene) in WHSV 9.1 hour⁻¹. The operation proceeds continuously for about 300 hours without observing appreciable variations in the catalyst behaviour. Reactant conversions and selectivity towards the useful reaction product are totally similar to those of Example 1.

## Claims

1. A catalytic process for preparing cumene by alkylating benzene with propylene, or by transalkylating diisopropylbenzenes in the presence of benzene, either reaction being catalyzed by an X-ray-amorphous silica-alumina gel having an SiO₂/Al₂O₃ of from 50:1 to 300:1, comprising the step of reacting benzene with propylene at a temperature of from 100°C to 250°C under a pressure of from 2 MPa to 5 Mpa (20 to 50 ata), the molar ratio of benzene to propylene being from 2:1 to 30:1 , or transalkylating diisopropylbenzenes in the presence of benzene at a temperature of from 150°C to 250°C under a pressure of from 1 Mpa to 4 MPa (10 to 40 ata), the molar ratio of benzene to the diisopropylbenzenes being from 10:1 to 40:1, in the presence of an X-ray-amorphous silica-alumina gel having a surface area of from 500 m²/g to 1000 m²/g, an overall pore volume of from 0,3 ml/g to 0, 6 ml/g, and a mean pore diameter of from 1 nm (10 A), said silica-alumina gel being free of pores having a diameter greater than 3 nm (30 A).

2. Process according to Claim 1, wherein the alkylation of benzene with propylene is carried out at a temperature of from 110°C to 200°C under a pressure of 3 MPa (30 ata), the molar ratio of benzene to propylene being from 4:1 to 15:1.

3. Process for preparing an X-ray-amorphous silica-alumina gel having a surface area of from 500 m²/g to 1000 m²/g, an overall pore volume of from 0,3 ml/g to 0, 6 ml/g, and a mean pore diameter of from 1 nm (10 A), said silica-alumina gel being free of pores having a diameter greater than 3 nm (30 A) for use as an alkylation or transalkylation catalyst, comprising the steps of:
(a) Preparing an aqueous solution of a tetraalkyl hydroxide (TAA-OH), a water soluble Al compound capable of being hydrolyzed to Al₂O₃ and a soluble Si compound capable of being hydrolyzed to SiO₂, the molar ratios of the solutes being:
SiO₂ to Al₂O₃: from 30:1 to 500:1
TAA-OH to SiO₂: from 0,005:1 to 0,2:1
H₂O to SiO₂ from 5:1 to 40:1;
(b) Heating said aqueous solution to gel it;
(c) Drying the as-obtained gel, and
(d) Calcining the dried gel initially in an inert atmosphere and finally in an oxidizing atmosphere.

4. Process according to Claim 3, wherein the molar ratios of the solutes are:
SiO₂ to Al₂O₃: from 50:1 to 300:1
TAA-OH to SiO₂: from 0,005:1 to 0,2:1
H₂O to SiO₂ from 10:1 to 25:1.

5. Process according to Claim 3, wherein the alkyl in said tetraalkyl hydroxide (TAA-OH) is selected from ethyl, n.propyl and n.butyl.

## Patentansprüche

1. Katalytisches Verfahren zur Herstellung von Cumol durch Alkylieren von Benzol mit Propylen oder durch Transalkylieren von Diisopropylbenzolen in Gegenwart von Benzol, wobei beide Reaktionen durch ein röntgenstrahlen-amorphes Siliciumdioxid-Aluminiumoxidgel mit einem SiO₂/Al₂O₃ Verhältnis von 50:1 bis 300:1 katalysiert werden, dadurch **gekennzeichnet**, daß man in einer Stufe Benzol mit Propylen bei einer Temperatur von 100°C bis 250°C unter einem Druck von 2 MPa bis 5 MPa (20 bis 50 ata) umsetzt, wobei das Molverhältnis von Benzol zu Propylen 2:1 bis 30:1 beträgt, oder die Diisopropylbenzole in Gegenwart von Benzol bei einer Temperatur von 150°C bis 250°C unter einem Druck von 1 MPa bis 4 MPa (10 bis 40 ata) transalkyliert, wobei das Molverhältnis von Benzol zu den Diisopropylbenzolen 10:1 bis 40:1 betragt, wobei beide Reaktionen in Gegenwart eines röntgenstrahlen-amorphen Siliciumdioxid-Aluminiumoxidgels mit einer spezifischen Oberfläche von 500 m²/g bis 1000 m²/g, einem Gesamtporenvolumen von 0,3 ml/g bis 0,6 ml/g, und einem mittleren Porendurchmesser von 1 nm (10 Å) durchgeführt werden, wobei das Siliciumdioxid-Aluminiumoxidgel frei von Poren mit einem größeren Durchmesser als 3 nm (30 Å) ist.

2. Verfahren nach Anspruch 1, dadurch **gekennzeichnet**, daß man Benzol mit Propylen bei einer Temperatur von 110°C bis 200°C unter einem Druck von 3 MPa (30 ata) alkyliert, wobei das Molverhältnis von Benzol zu Propylen 4:1 bis 15:1 beträgt.

3. Verfahren zur Herstellung eines röntgenstrahlen-amorphen Siliciumdioxid-Aluminiumoxidgels mit einer spezifischen Oberfläche von 500 m²/g bis 1000 m²/g, einem Gesamtporenvolumen von 0,3 ml/g bis 0,6 ml/g und einem mittleren Porendurchmesser von 1 nm (10 Å), wobei das Siliciumdioxid-Aluminiumoxidgel frei von Poren mit einem größeren Durchmesser als 3 nm (30 Å) ist, zur Verwendung als Alkylierungs- oder Transalkylierungskatalysator, dadurch **gekennzeichnet**, daß man in Stufen
(a) eine wäßrige Lösung aus Tetraalkylhydroxid (TAA-OH), einer wasserlöslichen Al-Verbindung, die zu Al₂O₃ hydrolysiert werden kann, und einer löslichen Si-Verbindung, die zu SiO₂ hydrolysiert werden kann, herstellt, wobei die Molverhältnisse der gelösten Stoffe
SiO₂ zu Al₂O₃: von 30:1 bis 500:1
TAA-OH zu SiO₂: von 0,005:1 bis 0,2:1
H₂O zu SiO₂ : von 5:1 bis 40:1
betragen;
(b) die wässrige Lösung zur Gelbildung erhitzt;
(c) das so erhaltene Gel trocknet; und
(d) das getrocknete Gel zuerst in einer Inertatmosphäre und abschließend in einer oxidierenden Atmosphäre calcimiert.

4. Verfahren nach Anspruch 3, dadurch **gekennzeichnet**, daß die Molverhältnisse der gelösten Stoffe
SiO₂ zu Al₂O₃: von 50:1 bis 300:1
TAA-OH zu SiO₂: von 0,0005:1 bis 0,2:1
H₂O zu SiO₂: von 10:1 bis 25:1
betragen.

5. Verfahren nach Anspruch 3, dadurch **gekennzeichnet**, daß der Alkylrest in dem Tetraalkylhydroxid (TAA-OH) aus Ethyl, n-Propyl und n-Butyl ausgewählt ist.

## Revendications

1. Procédé catalytique pour la préparation de cumène par alkylation du benzène avec du propylène ou par transalkylation de diisopropylbenzène en présence de benzène, ces réactions étant catalysées par un gel de silice-alumine amorphe au rayon X ayant un rapport SiO₂,/Al₂O₃ de 50:1 à 300:1, comprenant l'étape de réaction du benzène avec le propylène à une température de 100 à 250°C sous une pression de 2 MPa à 5 MPa (20 à 50 ata), le rapport molaire de benzène sur le propylène étant de 2:1 à 30:1, ou l'étape de transalkylation de diisopropylbenzènes en présence de benzène à une température de 150 à 250°C sous une pression de 1 MPa à 4 MPa (10 à 40 ata), le rapport molaire de benzène sur les diisopropylbenzènes étant de 10:1 à 40:1, en présence d'un gel de silice-alumine amorphe au rayon X ayant une surface spécifique de 500 m²/g à 1000 m²/g, et un volume de pore de 0,3 ml/g à 0,6 ml/g, et un diamètre moyen de pore de 1 nm (10 A), ledit gel de silice-alumine étant exempt de pore ayant un diamètre supérieur à 3 nm (30 A).

2. Procédé selon la revendication 1, caractérisé en ce que l'alkylation du benzène avec le propylène est effectuée à une température de 110 à 200°C sous une pression de 30 MPa (30 ata), le rapport molaire de benzène sur le propylène étant de 4:1 à 15:1.

3. Procédé pour la préparation d'un gel silice-alumine amorphe au rayon X ayant une surface spécifique de 500 m²/g à 1000 m²/g, un volume total de pore de 0,3 ml/g à 0,6 ml/g, et un diamètre moyen de pore de 1 nm (10 A), ledit gel de silice-alumine étant exempt de pore ayant un diamètre supérieur à 3 nm (30 A) pour son utilisation à titre de catalyseur d'alkylation ou de transalkylation, comprenant les étapes suivantes :
(a) préparation d'une solution aqueuse d'un hydroxyde de tétraalkyle (TAA-OH), d'un composé d'aluminium soluble dans l'eau capable d'être hydrolysé en Al₂O₃ et d'un composé soluble de silice capable d'être hydrolysé en SiO₂, le rapport molaire des solutés étant :
SiO₂ sur Al₂O₃ : de 30:1 à 500:1
TAA-OH sur SiO₂ : de 0,005:1 à 0,2:1
H₂O sur SiO₂ : de 5:1 à 40:1 ;
(b) chauffage de ladite solution aqueuse jusqu'à obtention d'un gel ;
(c) séchage du gel ainsi obtenu, et
(d) calcination du gel séché, initialement dans une atmosphère inerte et pour finir en atmosphère oxydante.

4. Procédé selon la revendication 3, caractérisé en ce que les rapports molaires des solutés sont :
SiO₂ sur Al₂O : de 50:1 à 300:1
TAA-OH sur SiO 2 : de 0,005:1 à 0,2:1
H₂O sur SiO₂ : de 10:1 à 25:1.

5. Procédé selon la revendication 3, caractérisé en ce que l'alkyle dans l'hydroxyde de tétraalkyle (TAA-OH) est choisi parmi l'éthyle, le N-propyle et le N-butyle.
